# EUROPÄISCHE PATENTSCHRIFT

(11) **EP 1 515 085 B1**
(45) Veröffentlichungstag und Bekanntmachung des Hinweises auf die Patenterteilung: **24.06.2015**
(21) Anmeldenummer: 04021097.3
(22) Anmeldetag: 06.09.2004
(51) Int. Cl.: F21V 17/10, A61N 5/06, F21V 17/06, F21V 27/02, F21V 17/14, F21V 19/00

(54) **UV-Lampenanordnung und deren Verwendung**
UV lamp assembly and use thereof
Ensemble lampe UV et son utilisation

(30) Priorität: 15.09.2003 DE 10342876; 01.10.2003 DE 10346131
(43) Veröffentlichungstag der Anmeldung: 16.03.2005
(73) Patentinhaber: Heraeus Noblelight GmbH, 63450 Hanau (DE)
(72) Erfinder: Berger, Ulrich, 63599 Biebergemünd (DE); Greif, Stefan, 36039 Fulda (DE); Smolka, Ernst Dr, 63773 Goldbach (DE); Lang, Jürgen, 60314 Frankfurt (DE); Ullrich, Bernd, 63526 Erlensee (DE)
(74) Vertreter: Kühn, Hans-Christian

(56) Entgegenhaltungen:
- EP-A2- 1 306 106
- DE-C- 445 204
- DE-U1- 8 907 132
- DE-U1- 9 001 445
- GB-A- 1 475 663

## Beschreibung

Die Erfindung betrifft eine UV-Lampenanordnung mit einem Lampensockel, mit einem am Lampensockel angeordneten, konkaven Reflektorkörper, mit einem innerhalb der Kavität des Reflektorkörpers und am Lampensockel angeordneten UV-Brenner, insbesondere mit einer Filterscheibe, die eine Austrittsöffnung des Reflektorkörpers für UV-Strahlung bedeckt. Die Erfindung betrifft weiterhin eine Verwendung dieser UV-Lampenanordnung.

Derartige Lampenanordnungen sind aus der WO 97/32158 für Besonnungsgeräte bekannt. In einem Glaskolben, der innen mit einer Reflexionsschicht belegt ist, ist hier eine Quecksilberdampfhochdrucklampe stehend als Bräunungsstrahler angeordnet. Der Glaskolben wird von einem UV-Filter abgeschlossen. Dabei ist der Filter und/oder der Bräunungsstrahler austauschbar. Nachdem bei der hier offenbarten Lampenanordnung der Glaskolben zum Bräunungsstrahler und zum UV-Filter mittels Dichtungen luftdicht abgeschlossen werden soll, um den Glaskoben evakuieren zu können, erweist sich ein Wechsel des UV-Filters oder des Strahlers als problematisch bezüglich einer erneuten Abdichtung.

Die EP 301 208 offenbart einen Bajonettsockel für eine Lampe oder einen Reflektor, wobei der Bajonettverschluss den Lampensockel in einer Lampenfassung fixiert. Zwischen dem Reflektor und dem Bajonettsockel sind Steck- oder Klemmverbindungen offenbart, wobei der Reflektor vom Sockel lösbar sein kann.

Die DE 20106885U1 offenbart ebenfalls eine Lampe mit einem Bajonettsockel, wobei der Bajonettverschluss den Sockel in einer Lampenfassung fixiert. Die Lampe weist einen vom Bajonettsockel abschraubbaren Lampenkolben auf.

DE 445204 C ist der nächstliegende Stand der Technik.

Es ist nun Aufgabe der Erfindung, ein UV-Lampensystem der eingangs genannten Art bereitzustellen, bei dem der Austausch des Bräunungsstrahlers einfacher und problemloser ausgeführt werden kann, wobei die Weiterverwendung der übrigen Teile des UV-Lampensystems weiterhin ermöglicht wird.

Die Aufgabe wird dadurch gelöst, dass der Reflektorkörper über einen Bajonettverschluss lösbar mit dem Lampensockel verbunden ist. Durch eine derartige Ausgestaltung der Lampenanordnung kann der Reflektorkörper ggf. inklusive einer Filterglasscheibe durch einen einfachen Handgriff abgenommen werden, so dass der Brenner direkt zugänglich ist. Es ist nun möglich, entweder den Brenner allein auszutauschen oder diesen inklusive Sockel zu ersetzen. Der Reflektorkörper und eine ggf. daran angeordnete Filterscheibe können weiterverwendet werden.

Die Aufgabe wird auch dadurch gelöst, dass der Reflektorkörper über mindestens zwei Greifarme mit dem Lampensockel verbunden ist. Eine derartige Ausgestaltung ermöglicht wie der Bajonettverschluss einen einfachen Austausch des UV-Brenners bei dessen Ausfall und eine Wiederverwendung aller restlichen Bauteile wie Reflektor, Filterglasscheibe, Lampensockel, elektrischen Anschlüssen und Greifarmen.

Die erfindungsgemäße Lösung ist für alle Lampenanordnungen, die für optische Strahlung vorgesehen sind, geeignet. Hierzu kann optische Strahlung mit einem Strahler erzeugt werden. Hat der Strahler sein Maximum im VIS oder IR, handelt es sich um eine VIS- oder IR-Lampenanordnung mit einem VIS- oder IR-Brenner, deren Reflektor vorzugsweise eine VISoder IR-reflektierende Schicht aufweist. Der Reflektor kann dabei ggf. für UV-Strahlung durchlässig sein. Insbesondere ist die Lampenanordnung für UV-Strahlung vorgesehen, die mit einem UV-Strahler erzeugt wird.

Dabei hat es sich bewährt, wenn der Reflektorkörper einen Reflektorhals aufweist und der Reflektorhals über den Bajonettverschluss lösbar mit dem Lampensockel verbunden ist, oder aber wenn der Reflektorkörper an der Strahleröffnung des Reflektors über einen Bajonettverschluss oder mit Greifarmenlösbar mit dem Lampensockel verbunden ist.

Es ist bevorzugt, den Reflektorkörper aus Glas, Glaskeramik, Keramik oder Metall zu bilden. Der Reflektorkörper weist dabei vorzugsweise eine UV-Strahlung reflektierende Beschichtung auf.

Insbesondere hat es sich bewährt, wenn der Reflektorkörper aus Glas oder Glaskeramik gebildet ist und seine Beschichtung für Infrarotstrahlung durchlässig ist. So wird Wärme aus dem Reflektorkörper abtransportiert und eine Kühlung des Reflektorkörpers kann unterbleiben.

Es hat sich bewährt, wenn der Reflektorkörper Facetten aufweist.
Weiterhin hat es sich bewährt, wenn die UV-Strahlung reflektierende Beschichtung des Reflektorkörpers mindestens ein Metalloxid enthält.

Es ist von Vorteil, wenn die Filterscheibe mit dem Reflektorkörper durch einen elastischen Kleber verbunden ist. Vorzugsweise wird ein Kleber auf Silikonbasis gewählt

Dabei ist bevorzugt, dass die Filterscheibe derart mit dem Reflektorkörper verbunden ist, dass Öffnungen verbleiben, über welche ein Luftaustausch zwischen einem Raum außerhalb und dem Raum innerhalb des Reflektorkörpers erfolgen kann.
Dabei hat es sich bewährt, wenn der Reflektorkörper im Bereich der Austrittsöffnung an seinem Umfang mindestens eine halbmondförmige Aussparung aufweist.

Es ist aber genauso möglich, dass die Filterscheibe die Austrittsöffnung des Reflektorkörpers vollständig verschließt.

Es hat sich bewährt, wenn der Lampensockel aus Keramik, Kunststoff oder Metall gebildet ist. Dabei wird Keramik besonders bevorzugt, da es nicht nur hoch temperaturbeständig sondern auch elektrisch isolierend ist, so dass die elektrischen Anschlüsse für den UV-Brenner nicht gesondert elektrisch vom Sockel isoliert werden müssen.

Es hat sich weiterhin als günstig erwiesen, wenn der Reflektorkörper aus Borosilikatglas oder Kalk-Natron-Glas gebildet ist.

Besonders bevorzugt ist es, wenn der Reflektorhals und der Reflektorkörper aus demselben Material und in einem Stück ausgebildet sind, wobei am Reflektorhals Noppen angeformt sind, so dass er direkt mit dem Lampensockel verbunden werden kann.

Es ist aber genauso möglich, dass der Reflektorhals und der Reflektorkörper aus demselben Material und in einem Stück ausgebildet sind, wobei der Reflektorhals mit einem als Ring ausgestaltetem weiteren Bauteil verbunden ist, an welchem Noppen angeformt sind, so dass der Ring direkt mit dem Lampensockel verbunden werden kann. Dabei kann der Ring aus Metall, Kunststoff oder Keramik gebildet sein.
Es hat sich bewährt, wenn der Ring mit dem Reflektorhals mittels Glaslot oder Kleber verbunden ist oder der Ring auf den Reflektorhals aufgeschrumpft ist. Hierzu sind sowohl Ein- als auch Mehrkomponentenkleber geeignet. Insbesondere ist es bevorzugt, wenn der Kleber ein anorganischer Kleber ist, wie beispielsweise Sauereisenzement oder wenn der Kleber auf Silikonbasis ist.

Besonders günstig ist es, wenn die Greifarme aus gebogenem Draht oder gebogenen Blechstreifen gebildet sind.

Es ist bevorzugt, einen Metallhalogenidstrahler als UV-Brenner einzusetzen.
Dabei weist der UV-Brenner vorzugsweise einen Lampenkolben aus Kieselglas sowie elektrische Kontakte auf, die entweder an einer Seite oder an sich gegenüber liegenden Seiten des Lampenkolbens durch diesen gasdicht hindurchgeführt sind.

Eine Verwendung der erfindungsgemäßen UV-Lampenanordnung ohne Zwangskühlung in einem Leistungsbereich von 100W bis 300W ist ideal.

Besonders bevorzugt ist dabei die Verwendung von mindestens einer UV-Lampenanordnung in einem Bräunungsgerät.
Dabei hat es sich besonders bewährt, wenn die mindestens eine UV-Lampenanordnung mittels zwei Halteschrauben, die in einem Abstand von 25 - 27mm zueinander angeordnet sind, in dem Bräunungsgerät montiert ist.

Weiterhin hat es sich bewährt, wenn die mindestens eine UV-Lampenanordnung mittels zwei Halteschrauben, die in einem Abstand von 29 - 31mm zueinander angeordnet sind, in dem Bräunungsgerät montiert ist.

Die Figuren 7 bis 12 sollen die erfindungsgemäße UV-Lampenanordnung beispielhaft erläutem. Dabei betreffen Figuren 1 bis 6b Ausführungen mit Bajonettverschluss, und Figuren 7 bis 12 Ausführungen mit Greifarmen. So zeigt/zeigen:
- Fig. 1a bis 1e: eine UV-Lampenanordnung mit rechteckigem Lampensockel in unterschiedlichen Ansichten;
- Fig. 2a bis 2e: eine weitere UV-Lampenanordnung mit rundem Lampensockel in unterschiedlichen Ansichten;
- Fig. 3a bis 3e: eine weitere UV-Lampenanordnung mit rundem Lampensockel in unterschiedlichen Ansichten;
- Fig. 4a bis 4f: eine weitere UV-Lampenanordnung mit rundem Lampensockel in unterschiedlichen Ansichten, wobei nur eine Hälfte des Lampensockels dargestellt ist;
- Fig. 5a bis 5e: eine weitere UV-Lampenanordnung mit rundem Lampensockel in unterschiedlichen Ansichten;
- Fig. 6a bis 6b: eine weitere UV-Lampenanordnung mit länglichen Lampensockel mit und ohne Reflektor;
- Fig. 7: eine dreidimensionale Ansicht von zwei UV-Lampenanordnungen mit je vier Greifarmen aus gebogenen Blechstreifen;
- Fig. 8: eine weitere dreidimensionale Ansicht der UV-Lampenanordnungen aus Fig. 7;
- Fig. 9: eine Ausführung bei der die Greifarme so ausgebildet sind, dass sie den Sockel an der Strahlereintrittsöffnung des Reflektors mit Federspannkraft gegen den Reflektor drücken;
- Fig. 10: eine Lampenanordnung, bei der ein Sockel mit zwei Greifarmen am Reflektorkörper gehalten wird. Dabei sind die Greifarme so ausgebildet, dass sie den Sockel gegen den Reflektorhals klemmen;
- Fig. 11: eine Ausführung bei der die Greifarme als Klammern ausgebildet sind, die den Sockel gegen den Reflektor drücken, wobei die Greifarme in die Reflektoröffnung eingreifen;
- Fig. 12: eine Lampenanordnung, bei der ein Sockel mit zwei Greifarmen am Reflektorkörper gehalten wird. Dabei sind die Greifarme so ausgebildet, dass sie den Sockel gegen den Reflektorhals klemmen.

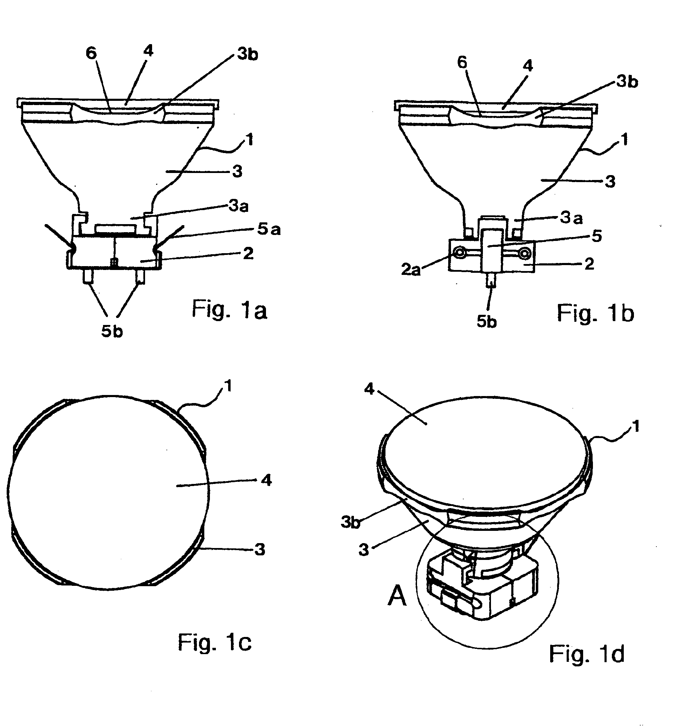

Figur 1a zeigt eine UV-Lampenanordnung 1 in der Seitenansicht mit einem zweiteiligen, rechteckigen Lampensockel 2 und einem Reflektorkörper 3 aus Glas. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, der über einen Bajonettverschluss direkt mit dem Lampensockel 2 verbunden ist. Außerdem sind im Bereich der Austrittsöffnung des Reflektorkörpers 3 für UV-Strahlung halbmondförmige Aussparungen 3b derart angeordnet, dass Öffnungen 6 einen Luftaustausch zum Raum im Reflektorkörper 3 ermöglichen. Am Lampensockel 2 sind eine Feder 5a sowie Halteschrauben 5b zum Einbau der UV-Lampenanordnung 1 in ein Bräunungsgerät vorhanden.

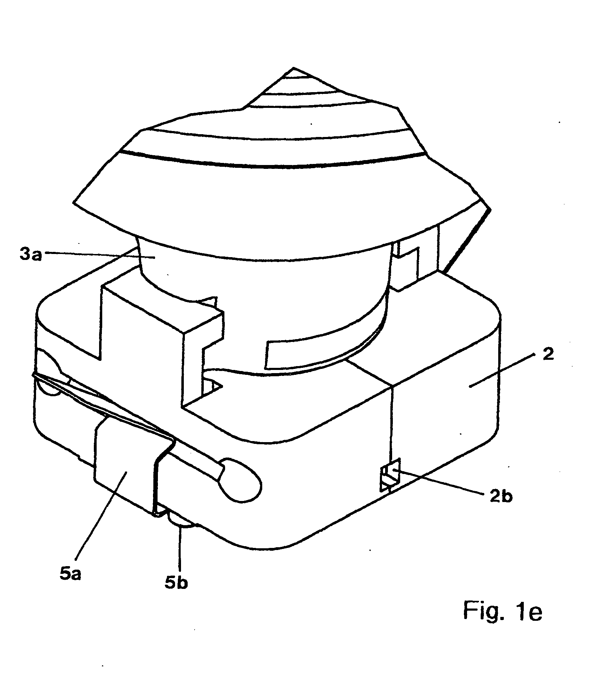

Figur 1b zeigt die UV-Lampenanordnung 1 aus Fig. 1 in einer weiteren Seitenansicht, allerdings um 90° gedreht. Es ist zu erkennen, dass die beiden Teile des zweiteiligen Lampensockels 2 durch Nieten oder Verschraubungen 2a verbunden sind.

Figur 1c zeigt die UV-Lampenanordnung 1 aus den Figuren 1a und 1b in der Draufsicht.

Figur 1d zeigt die UV-Lampenanordnung 1 aus den Figuren 1a bis 1c in dreidimensionaler Ansicht.

Figur 1e zeigt den Ausschnitt A aus Figur 1d in vergrößerter Darstellung. Dabei ist der Bajonettverschluss zwischen dem Reflektorkörper 3 und dem Lampensockel 2 deutlich zu erkennen. Weiterhin sind Öffnungen 2b im Lampensockel 2 vorhanden, durch welche zu beiden Seiten des Lampensockels 2 elektrische Anschlüsse eines hier nicht zu sehenden, im Reflektorkörper 3 angeordneten UV-Brenners geführt werden.

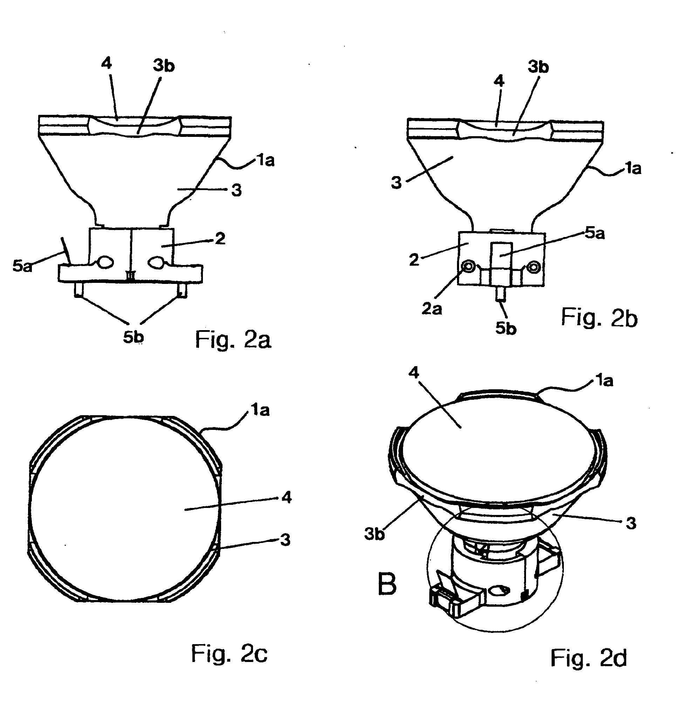

Figur 2a zeigt eine weitere UV-Lampenanordnung 1a in der Seitenansicht mit einem zweiteiligen, runden Lampensockel 2 und einem Reflektorkörper 3 aus Glas. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, der über einen Bajonettverschluss direkt mit dem Lampensockel 2 verbunden ist. Außerdem sind im Bereich der Austrittsöffnung des Reflektorkörpers 3 für UV-Strahlung halbmondförmige Aussparungen 3b angeordnet. Allerdings bedeckt hier die Filterscheibe 4 den Reflektorkörper 3 vollständig, so dass keine Öffnung verbleibt. Am Lampensockel 2 sind eine Feder 5a sowie Halteschrauben 5b zum Einbau der UV-Lampenanordnung 1a in ein Bräunungsgerät vorhanden.

Figur 2b zeigt die UV-Lampenanordnung 1a aus Fig. 2a in einer weiteren Seitenansicht, allerdings um 90° gedreht. Es ist zu erkennen, dass die beiden Teile des zweiteiligen Lampensockels 2 durch Nieten oder Verschraubungen 2a verbunden sind.

Figur 2c zeigt die UV-Lampenanordnung 1a aus den Figuren 2a und 2b in der Draufsicht.

Figur 2d zeigt die UV-Lampenanordnung 1a aus den Figuren 2a bis 2c in dreidimensionaler Ansicht.

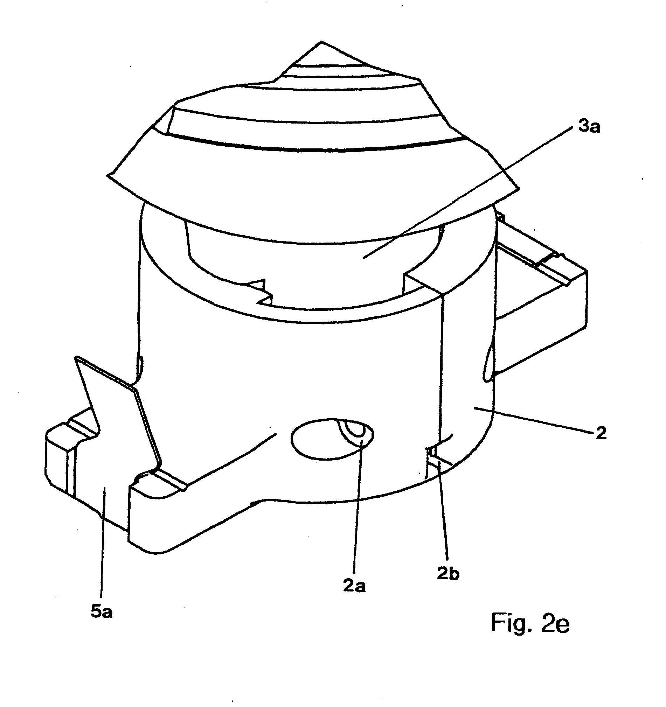

Figur 2e zeigt den Ausschnitt B aus Figur 2d in vergrößerter Darstellung. Dabei ist der Bajonettverschluss zwischen dem Reflektorkörper 3 und dem Lampensockel 2 deutlich zu erkennen. Weiterhin sind Öffnungen 2b im Lampensockel 2 vorhanden, durch welche zu beiden Seiten des Lampensockels 2 elektrische Anschlüsse eines hier nicht zu sehenden, im Reflektorkörper 3 angeordneten UV-Brenners geführt werden.

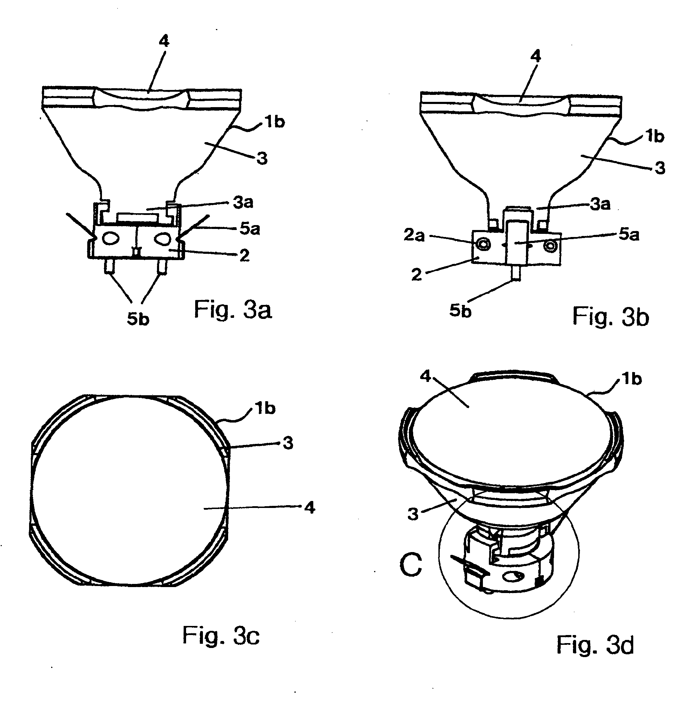

Figur 3a zeigt eine UV-Lampenanordnung 1 b in der Seitenansicht mit einem zweiteiligen, runden Lampensockel 2 und einem Reflektorkörper 3 aus Glas. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, der über einen Bajonettverschluss direkt mit dem Lampensockel 2 verbunden ist. Außerdem sind im Bereich der Austrittsöffnung des Reflektorkörpers 3 für UV-Strahlung halbmondförmige Aussparungen 3b angeordnet. Allerdings bedeckt hier die Filterscheibe 4 den Reflektorkörper 3 vollständig, so dass keine Öffnung verbleibt. Am Lampensockel 2 sind eine Feder 5a sowie Halteschrauben 5b zum Einbau der UV-Lampenanordnung 1 b in ein Bräunungsgerät vorhanden.

Figur 3b zeigt die UV-Lampenanordnung 1 b aus Fig. 3a in einer weiteren Seitenansicht, allerdings um 90° gedreht. Es ist zu erkennen, dass die beiden Teile des zweiteiligen Lampensockels 2 durch Nieten oder Verschraubungen 2a verbunden sind.

Figur 3c zeigt die UV-Lampenanordnung 1 b aus den Figuren 3a und 3b in der Draufsicht.

Figur 3d zeigt die UV-Lampenanordnung 1 b aus den Figuren 3a bis 3c in dreidimensionaler Ansicht.

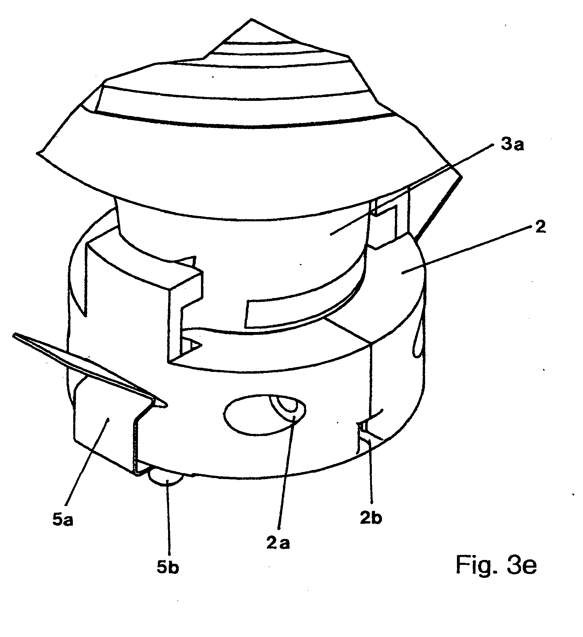

Figur 3e zeigt den Ausschnitt C aus Figur 3d in vergrößerter Darstellung. Dabei ist der Bajonettverschluss zwischen dem Reflektorkörper 3 und dem Lampensockel 2 deutlich zu erkennen. Weiterhin sind Öffnungen 2b im Lampensockel 2 vorhanden, durch welche zu beiden Seiten des Lampensockels 2 elektrische Anschlüsse eines hier nicht zu sehenden, im Reflektorkörper 3 angeordneten UV-Brenners geführt werden.

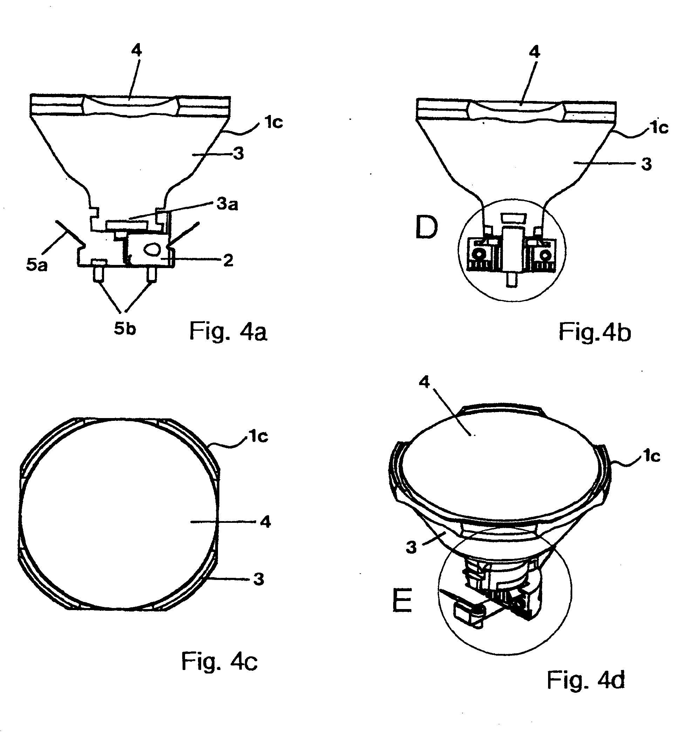

Figur 4a zeigt eine UV-Lampenanordnung 1 c in der Seitenansicht mit einem zweiteiligen, runden Lampensockel 2 und einem Reflektorkörper 3 aus Glas, wobei nur eine Hälfte des Lampensockels 2 dargestellt ist. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, der über einen Bajonettverschluss direkt mit dem Lampensockel 2 verbunden ist. Außerdem sind im Bereich der Austrittsöffnung des Reflektorkörpers 3 für UV-Strahlung halbmondförmige Aussparungen 3b angeordnet. Allerdings bedeckt hier die Filterscheibe 4 den Reflektorkörper 3 vollständig, so dass keine Öffnung verbleibt. Am Lampensockel 2 sind eine Feder 5a sowie Halteschrauben 5b zum Einbau der UV-Lampenanordnung 1 c in ein Bräunungsgerät vorhanden.

Figur 4b zeigt die UV-Lampenanordnung 1 c aus Fig. 4a in einer weiteren Seitenansicht, allerdings um 90° gedreht. Es ist das Innere des Lampensockels 2 zu erkennen.

Figur 4c zeigt die UV-Lampenanordnung 1 c aus den Figuren 4a und 4b in der Draufsicht.

Figur 4d zeigt die UV-Lampenanordnung 1 c aus den Figuren 4a bis 4c in dreidimensionaler Ansicht.

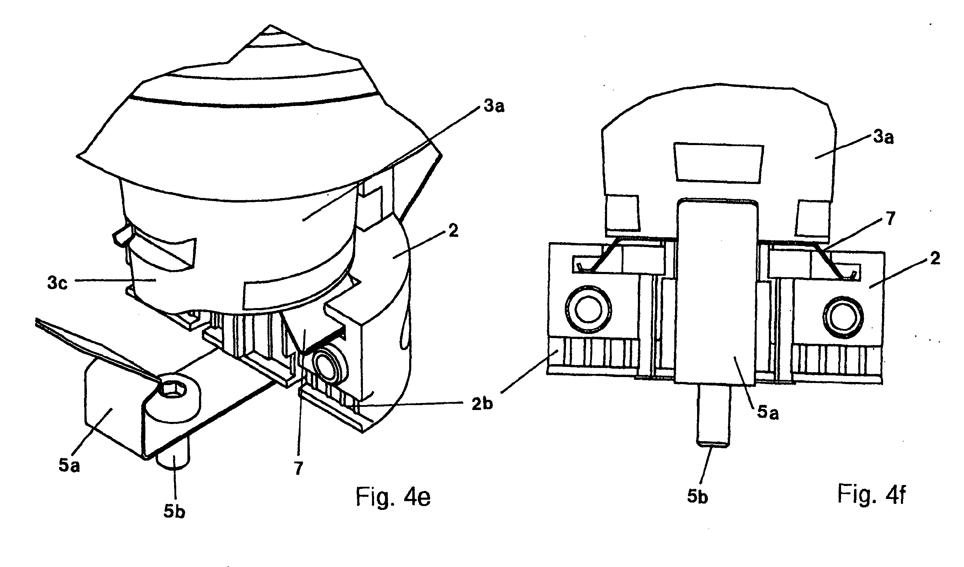

Figur 4e zeigt den Ausschnitt E aus Figur 4d in vergrößerter Darstellung. Dabei ist der Reflektorhals 3a mit daran angeformten Noppen 3c für den Bajonettverschluss deutlich zu erkennen. Weiterhin sind die Öffnungen 2b im Lampensockel 2 erkennbar, durch welche zu beiden Seiten des Lampensockels 2 elektrische Anschlüsse eines hier nicht zu sehenden, im Reflektorkörper 3 angeordneten UV-Brenners geführt werden. Zur Fixierung des Reflektorhalses 3a im Lampensockel 2 ist eine Blattfeder 7 vorhanden, die den Reflektorkörper 3 nach oben drückt und so den Bajonettverschluss fixiert.

Figur 4f zeigt den Ausschnitt D aus Figur 4b in vergrößerter Darstellung. Wieder sind die Öffnungen 2b im Lampensockel 2 erkennbar, durch welche zu beiden Seiten des Lampensockels 2 elektrische Anschlüsse eines hier nicht zu sehenden, im Reflektorkörper 3 angeordneten UV-Brenners geführt werden. Zur Fixierung des Reflektorhalses 3a im Lampensockel 2 ist die Blattfeder 7 vorhanden, die den Reflektorkörper 3 nach oben drückt und so den Bajonettverschluss fixiert.

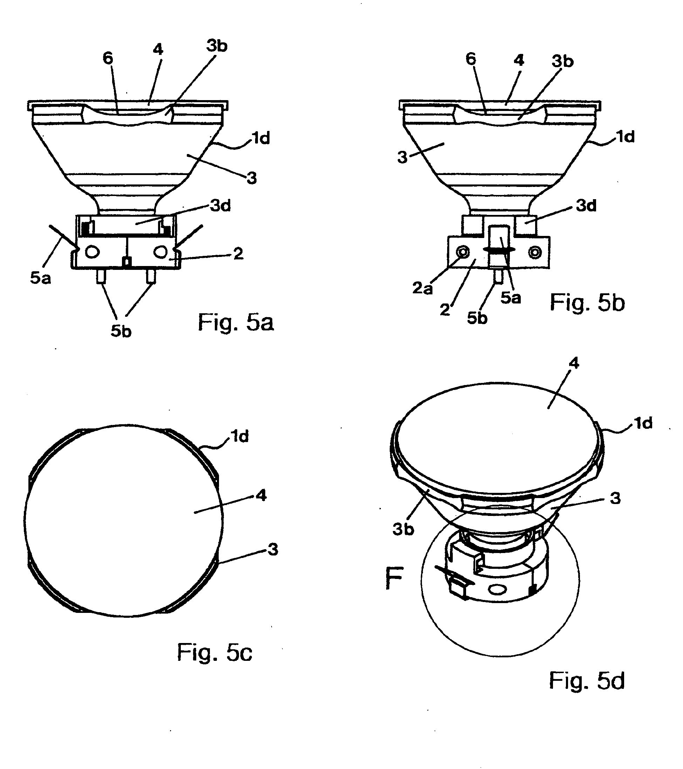

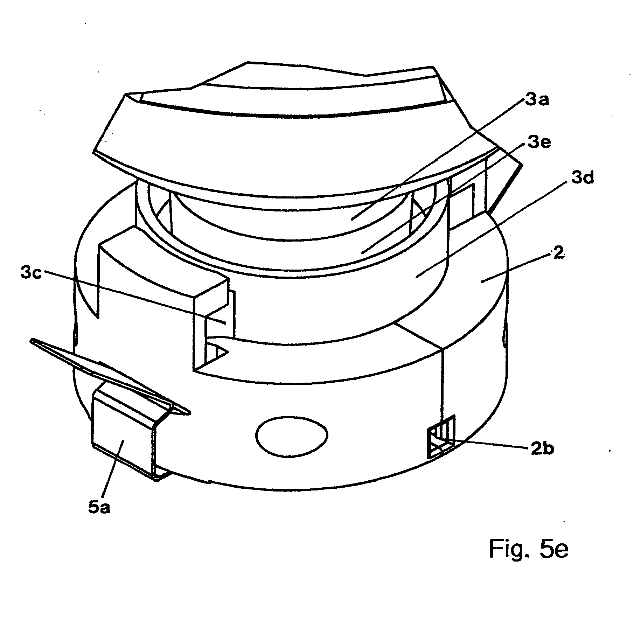

Figur 5a zeigt eine UV-Lampenanordnung 1 d in der Seitenansicht mit einem zweiteiligen, runden Lampensockel 2 und einem Reflektorkörper 3 aus Glas. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, an welchem ein als Ring ausgestaltetes weiteres Bauteil 3d angebracht ist. Das als Ring ausgestaltete Bauteil 3d weist dabei Noppen 3c auf ( siehe Fig. 5e) und ist über einen Bajonettverschluss direkt mit dem Lampensockel 2 verbunden. Außerdem sind im Bereich der Austrittsöffnung des Reflektorkörpers 3 für UV-Strahlung halbmondförmige Aussparungen 3b derart angeordnet, dass Öffnungen 6 einen Luftaustausch zum Raum im Reflektorkörper 3 ermöglichen. Am Lampensockel 2 sind eine Feder 5a sowie Halteschrauben 5b zum Einbau der UV-Lampenanordnung 1d in ein Bräunungsgerät vorhanden.

Figur 5b zeigt die UV-Lampenanordnung 1 d aus Fig. 5a in einer weiteren Seitenansicht, allerdings um 90° gedreht. Es ist zu erkennen, dass die beiden Teile des zweiteiligen Lampensockels 2 durch Nieten oder Verschraubungen 2a verbunden sind.

Figur 5c zeigt die UV-Lampenanordnung 1d aus den Figuren 5a und 5b in der Draufsicht.

Figur 5d zeigt die UV-Lampenanordnung 1 d aus den Figuren 5a bis 5c in dreidimensionaler Ansicht.

Figur 5e zeigt den Ausschnitt F aus Figur 5d in vergrößerter Darstellung. Dabei ist der Bajonettverschluss zwischen dem als Ring ausgestaltetem Bauteil 3d und dem Lampensockel 2 deutlich zu erkennen. Der Reflektorhals 3a ist mit dem als Ring ausgestaltetem Bauteil 3d im Bereich 3e mittels eines hier nicht dargestellten Klebers fest verbunden. Weiterhin sind Öffnungen 2b im Lampensockel 2 vorhanden, durch welche zu beiden Seiten des Lampensockels 2 elektrische Anschlüsse eines hier nicht zu sehenden, im Reflektorkörper 3 angeordneten UV-Brenners geführt werden.

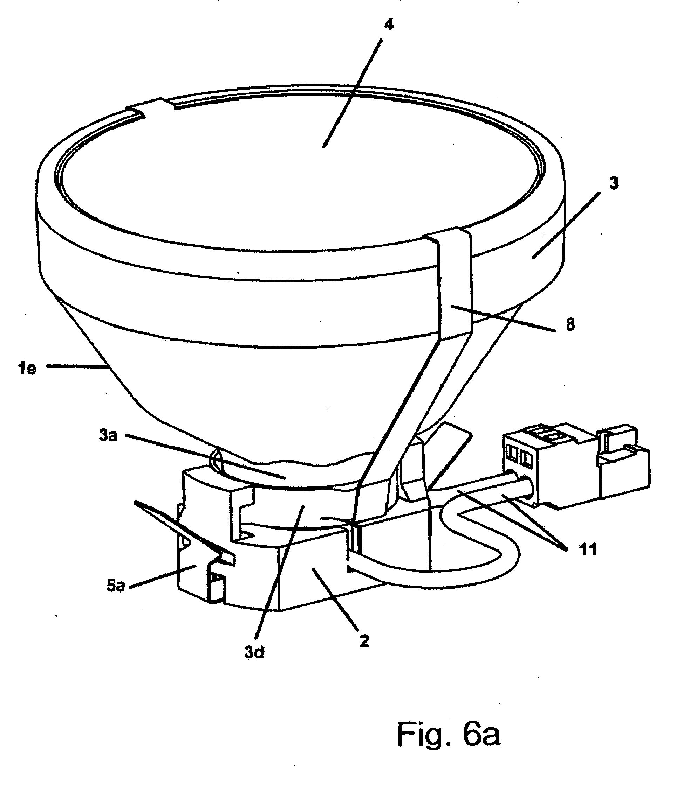

Figur 6a zeigt eine UV-Lampenanordnung 1 e in dreidimensionaler Ansicht mit einem einteiligen länglichen, an den kurzen Seiten abgerundeten Lampensockel 2 und einem Reflektorkörper 3 aus Glas. Die Austrittsöffnung des Reflektorkörpers 3 für UV-Strahlung ist mit einer Filterscheibe 4 bedeckt. Weiterhin weist der Reflektorkörper 3 einen Reflektorhals 3a auf, an welchem ein als Ring ausgestaltetes weiteres Bauteil 3d angebracht ist und über einen Bajonettverschluss direkt mit dem Lampensockel 2 verbunden ist. Zwei als Greifarme 8 ausgebildete Bügel stellen hier eine mechanische Verbindung zwischen dem Reflektorkörper 3 und dem Ring 3d dar. Am Lampensockel 2 sind eine Feder 5a zum Einbau der UV-Lampenanordnung 1 e in ein Bräunungsgerät vorhanden.

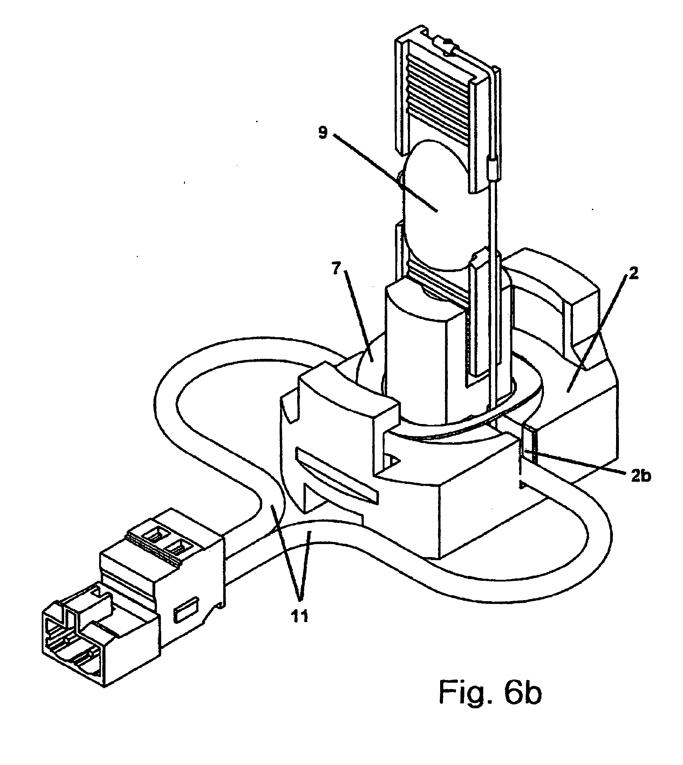

Figur 6b zeigt eine UV-Lampenanordnung aus Fig. 6a in dreidimensionaler Ansicht mit einem einteiligen, länglichen, an den Seiten abgerundeten Lampensockel 2 ohne einen Reflektorkörper 3 aus Glas. Deutlich zu erkennen ist der Brenner 9 in dem Lampensockel 2 mit der Blattfeder 7, die den Reflektorkörper 3 nach oben drückt und so den Bajonettverschluss fixiert. Im Lampensockel 2 sind die seitlichen Öffnungen 2b erkennbar, durch welche zu beiden Seiten des Lampensockels 2 die elektrischen Anschlüsse 11 des hier zu sehenden, im Reflektorkörper angeordneten UV-Brenners 9 geführt werden.

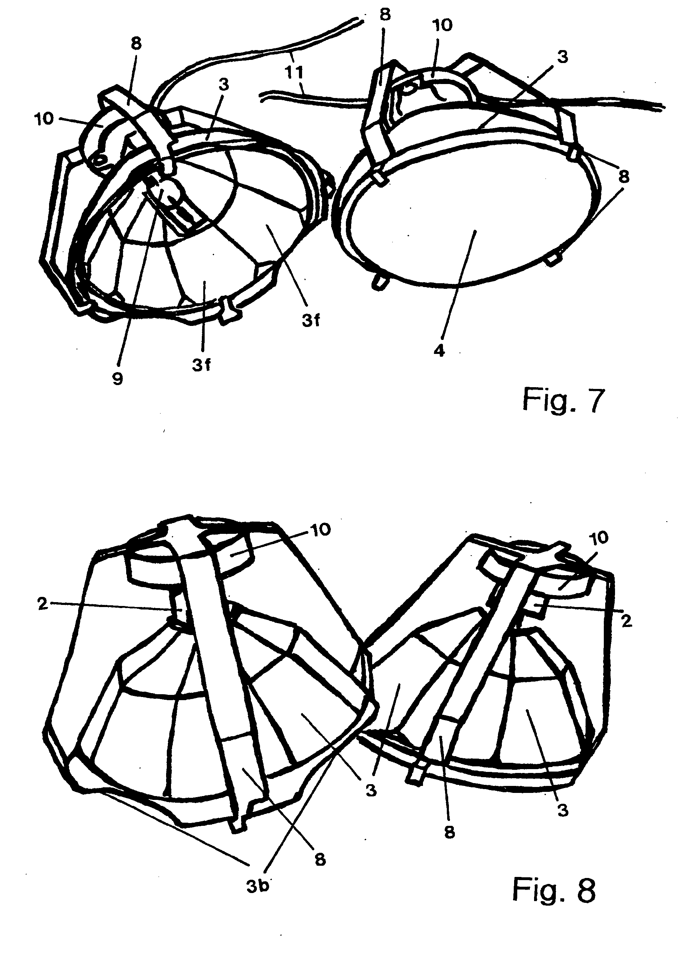

Figuren 7 und 8 zeigen dreidimensionale Ansichten von zwei UV-Lampenanordnungen 1, wobei jede einen Lampensockel 2 und einen konkaven Reflektorkörper 3 aufweist. Die rechte der beiden UV-Lampenanordnungen aus Fig. 7 zeigt eine Filterscheibe 4 am Reflektorkörper 3. Die linke der beiden UV-Lampenanordnungen aus Fig. 7 zeigt dagegen keine Filterscheibe 4 am Reflektorkörper 3, so dass der innerhalb der Kavität des Reflektorkörpers 3 angeordnete UV-Brenner 9 sichtbar wird. Die Halterung von Reflektorkörper 3 erfolgt über je vier Greifarme 8, die aus gebogenen Blechstreifen gebildet sind und eine einfache Demontage von Reflektorkörper 3 und Filterscheibe 4 und somit einen Austausch des UV-Brenners 9 ermöglichen. Der Reflektorkörper 3 ist mit Facetten 3f ausgestaltet. Die linke UV-Lampenanordnung 1 aus Fig. 8 zeigt weiterhin am Reflektorkörper 3 halbmondförmige Aussparungen 3b, die nach Montage einer Filterscheibe einen Luftaustausch zwischen dem Raum innerhalb des Reflektorkörpers 3 und dem Raum außerhalb des Reflektorkörpers 3 ermöglichen. Der elektrische Anschluss des UV-Brenners 9 mit dem Lampensockel 2 erfolgt über die Fassung 10 mit den elektrischen Anschlussdrähten 11.

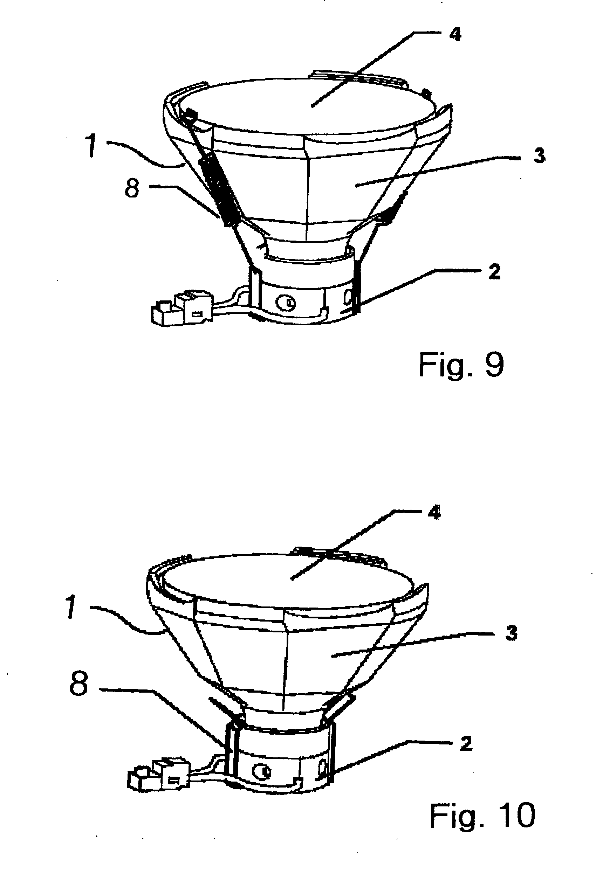

Figur 9 zeigt eine UV-Lampenanordnung 1 in dreidimensionaler Ansicht, in der die Greifarme 8 so ausgebildet sind, dass sie den Sockel 2 an der Reflektoraustrittsöffnung mit Federspannkraft gegen den Reflektor 3 drücken.

Figur 10 zeigt eine Lampenanordnung 1, bei der ein Sockel 2 mit zwei Greifarmen 8 am Reflektorkörper 3 gehalten wird. Dabei sind die Greifarme 8 so ausgebildet, dass sie den Sockel 2 gegen den Reflektorhals 3a oder das als Ring ausgestaltete Bauteil 3d klemmen.

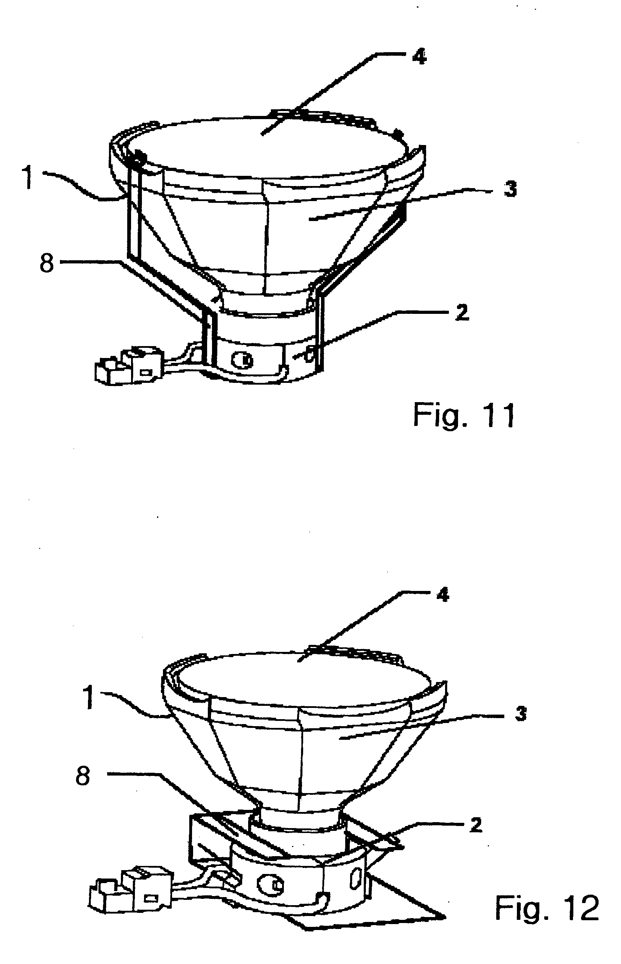

Figur 11 zeigt eine Lampenanordnung 1, in dreidimensonaler Darstellung, bei der die Greifarme 8, ähnlich wie in Fig. 9, als Klammem ausgebildet sind, die den Sockel gegen den Reflektor 3 drücken, wobei die Greifarme 8 in die Reflektoröffnung eingreifen.

Figur 12 zeigt eine Lampenanordnung 1, bei der ein Sockel 2 mit zwei Greifarmen 8 am Reflektorkörper 3 gehalten wird. Dabei sind die Greifarme 8 als U-Profil-Klammer ausgebildet, die den Sockel 2 gegen den Reflektorhals 3a oder ein als Ring ausgestaltetes Bauteil 3d klemmt.

## Patentansprüche

1. UV Lampenanordnung (1) mit einem Lampensockel (2), mit einem am Lampensockel (2) angeordneten Reflektorkörper (3), der eine Austrittsöffnung für optische Strahlung aufweist, und mit einem innerhalb des Reflektorkörpers (3) und am Lampensockel (2) angeordneten Brenner (9), wobei der Reflektorkörper (3) mit einer Filterscheibe bedeckt ist und über mindestens zwei als Klammern ausgebildeten Greifarme (8) mit dem Lampensockel (2) verbunden ist, die den Sockel (2) gegen den Reflektor (32) drücken **dadurch gekennzeichnet, dass** die Greifarme (8) in die Austrittsöffnung des Reflektorkörpers (3) eingreifen.

2. Lampenanordnung (1) nach Anspruch 1, **dadurch gekennzeichnet, dass** der Reflektorkörper (3) einen Reflektorhals (3a) aufweist.

3. Lampenanordnung (1) nach einem der Ansprüche 1 oder 2, **dadurch gekennzeichnet, dass** der Reflektorkörper (3) Facetten (3f) aufweist.

4. Lampenanordnung (1) nach einem der Ansprüche 1 bis 3, **dadurch gekennzeichnet, dass** der Reflektorhals (3a) und der Reflektorkörper (3) aus demselben Material und in einem Stück ausgebildet sind, wobei der Reflektorhals (3a) mit einem als Ring ausgestaltetem weiteren Bauteil (3d) verbunden ist, an welchem Noppen (3c) angeformt sind, so dass der Ring (3d) direkt mit dem Lampensockel (2) verbunden werden kann.

5. Lampenanordnung (1) nach einem der Ansprüche 1 bis 4, **dadurch gekennzeichnet, dass** die Greifarme (8) aus gebogenem Draht oder gebogenen Blechstreifen oder Kunststoff gebildet sind.

6. Lampenanordnung (1) nach einem der Ansprüche 1 bis 5, **dadurch gekennzeichnet, dass** der Reflektorkörper (3) mit einer Filterscheibe (4), die die Austrittsöffnung des Reflektorkörpers (3) für optische Strahlung bedeckt, verbunden ist.

7. Lampenanordnung (1) nach Anspruch 6, **dadurch gekennzeichnet, dass** die Filterscheibe (4) mit dem Reflektorkörper (3) durch einen elastischen Kleber oder eine Klammerung verbunden ist.

8. Lampenanordnung (1) nach einem der Ansprüche 6 oder 7, **dadurch gekennzeichnet, dass** die Filterscheibe (4) die Austrittsöffnung des Reflektorkörpers (3) vollständig verschließt.

9. Lampenanordnung (1) nach einem der Ansprüche 1 bis 8, **dadurch gekennzeichnet, dass** die Lampenanordnung eine UV-Lampenanordnung ist und der Brenner (9) ein UV-Brenner ist.

10. UV-Lampenanordnung (1) nach Anspruch 9, **dadurch gekennzeichnet, dass** der Reflektorkörper (3) eine UV-Strahlung reflektierende Beschichtung aufweist oder dass der Reflektorkörper (3) aus Glas oder Glaskeramik gebildet ist und dass seine Beschichtung für Infrarotstrahlung durchlässig ist.

11. UV-Lampenanordnung (1) nach einem der Ansprüche 9 oder 10, **dadurch gekennzeichnet, dass** der UV-Brenner (9) ein Metallhalogenidstrahler ist.

12. Lampenanordnung (1) nach einem der Ansprüche 4 bis 11, **dadurch gekennzeichnet, dass** der Ring (3d) mit dem Reflektorhals (3a) mittels Glaslot oder Kleber verbunden ist oder dass der Ring (3d) auf den Reflektorhals (3a) aufgeschrumpft ist.

13. Verwendung einer Lampenanordnung (1) nach einem der Ansprüche 1 bis 12 ohne Zwangskühlung in einem Leistungsbereich von 100W bis 300W.

14. Verwendung nach Anspruch 13 von mindestens einer UV-Lampenanordnung (1) in einem Bräunungsgerät.

## Claims

1. UV-lamp arrangement (1) having a lamp socket (2), having a reflector body (3) arranged on the lamp socket (2) that comprises an exit opening for optical radiation, and having a burner (9) that is arranged inside the reflector body (3) and on the lamp socket (2), whereby the reflector body (3) is A1 covered by a filter disk and is connected to the lamp socket (2), which is provided in the form of clamps, by means of at least two gripping arms (8) that push the socket (2) against the reflector (32), **characterised in that** the gripping arms (8) engage the exit opening of the reflector body (3).

2. Lamp arrangement (1) according to claim 1, **characterised in that** the reflector body (3) comprises a reflector neck (3a).

3. Lamp arrangement (1) according to either one of the claims 1 or 2, **characterised in that** the reflector body (3) comprises facets (3f).

4. Lamp arrangement (1) according to any one of the claims 1 to 3, **characterised in that** the reflector neck (3a) and the reflector body (3) are provided to be made of the same material and in the form of a single part, whereby the reflector neck (3a) is connected to a further component (3d) that is provided as a ring and has nubs (3c) formed to it such that the ring (3d) can be connected directly to the lamp socket (2).

5. Lamp arrangement (1) according to any one of the claims 1 to 4, **characterised in that** the gripping arms (8) are formed from curved wire or curved strips of sheet metal or plastics.

6. Lamp arrangement (1) according to any one of the claims 1 to 5, **characterised in that** the reflector body (3) is connected to a filter disk (4) that covers the exit opening of the reflector body (3) for optical radiation.

7. Lamp arrangement (1) according to claim 6, **characterised in that** the filter disk (4) is connected to the reflector body (3) by means of an elastic adhesive or a clamping.

8. Lamp arrangement (1) according to either one of the claims 6 or 7, **characterised in that** the filter disk (4) completely closes the exit opening of the reflector body (3).

9. Lamp arrangement (1) according to any one of the claims 1 to 8, **characterised in that** the lamp arrangement is an UV-lamp arrangement and the burner (9) is a UV-burner.

10. UV-lamp arrangement (1) according to claim 9, **characterised in that** the reflector body (3) comprises a UV-radiation-reflecting coating or **in that** the reflector body (3) is formed from glass or glass ceramics, and **in that** its coating is translucent for infrared radiation.

11. UV-lamp arrangement (1) according to either one of the claims 9 or 10, **characterised in that** the UV-burner (9) is a metal halide radiator.

12. Lamp arrangement (1) according to any one of the claims 4 to 11, **characterised in that** the ring (3d) is connected to the reflector neck (3a) by means of glass solder or adhesive or **in that** the ring (3d) is shrunk onto the reflector neck (3a).

13. Use of a lamp arrangement (1) according to any one of the claims 1 to 12 without forced cooling in a power range of 100 W to 300 W.

14. Use according to claim 13 of at least one UV-lamp arrangement (1) in a tanning device.

## Revendications

1. Agencement de lampe UV (1) comprenant un socle de lampe (2), un corps de réflecteur (3) agencé sur le socle de lampe (2), qui présente une ouverture de sortie pour le rayonnement optique, et un brûleur (9) disposé à l'intérieur du corps de réflecteur (3) et sur le socle de lampe (2), sachant que le corps de réflecteur (3) est couvert par un disque filtrant et est relié au socle de lampe (2) par au moins deux bras de préhension (8) formés en tant que pinces, qui appuient le socle (2) contre le réflecteur (32), **caractérisé en ce que** les bras de préhension (8) se mettent en prise dans l'ouverture de sortie du corps de réflecteur (3).

2. Agencement de lampe (1) selon la revendication 1, **caractérisé en ce que** le corps de réflecteur (3) présente un col de réflecteur (3a).

3. Agencement de lampe (1) selon la revendication 1 ou 2, **caractérisé en ce que** le corps de réflecteur (3) présente des facettes (3f).

4. Agencement de lampe (1) selon l'une des revendications 1 à 3, **caractérisé en ce que** le col de réflecteur (3a) et le corps de réflecteur (3) sont conçus dans le même matériau et d'un seul tenant, sachant que le col de réflecteur (3a) est relié à une pièce (3d) formée en tant que bague, sur laquelle des picots (3c) sont formés, de sorte que la bague (3d) peut être reliée directement au socle de lampe (2).

5. Agencement de lampe (1) selon l'une des revendications 1 à 4, **caractérisé en ce que** les bras de préhension (8) sont formés d'un fil métallique coudé ou d'une bande de tôle ou bien de plastique coudé(e).

6. Agencement de lampe (1) selon l'une des revendications 1 à 5, **caractérisé en ce que** le corps de réflecteur (3) est relié à un disque filtrant (4) qui couvre l'ouverture de sortie du corps de réflecteur (3) pour le rayonnement optique.

7. Agencement de lampe (1) selon la revendication 6, **caractérisé en ce que** le disque filtrant (4) est relié au corps de réflecteur (3) par un adhésif élastique ou une fixation.

8. Agencement de lampe (1) selon la revendication 6 ou 7, **caractérisé en ce que** le disque filtrant (4) ferme complètement l'ouverture de sortie du corps de réflecteur (3).

9. Agencement de lampe (1) selon l'une des revendications 1 à 8, **caractérisé en ce que** l'agencement de lampe est un agencement de lampe UV et le brûleur (9) est un brûleur UV.

10. Agencement de lampe UV (1) selon la revendication 9, **caractérisé en ce que** le corps de réflecteur (3) présente un revêtement réfléchissant le rayonnement UV ou que le corps de réflecteur (3) est conçu en verre ou en céramique de verre et que son revêtement laisse passer le rayonnement infrarouge.

11. Agencement de lampe UV (1) selon la revendication 9 ou 10, **caractérisé en ce que** le brûleur UV (9) est un spot d'halogénure métallique.

12. Agencement de lampe (1) selon l'une des revendications 4 à 11, **caractérisé en ce que** la bague (3d) est reliée au col de réflecteur (3a) via un brasage au verre ou un adhésif ou que la bague (3d) est emmanchée à chaud sur le col de réflecteur (3a).

13. Emploi d'un agencement de lampe (1) selon l'une des revendications 1 à 12 sans refroidissement forcé dans une plage de puissance de 100W à 300W.

14. Emploi selon la revendication 13 d'au moins un agencement de lampe UV (1) dans un appareil de brunissement.
